# EUROPEAN PATENT APPLICATION

(11) **EP 0 830 858 A1**
(43) Date of publication of application: **25.03.1998**
(21) Application number: 97307380.2
(22) Date of filing: 22.09.1997
(51) Int. Cl.: A61K 9/14, A61K 9/28, A61K 9/16, A61K 31/55

(54) **Formulation comprising coated olanzapine particles**

(30) Priority: 24.09.1996 US 26633 P
(71) Applicant: ELI LILLY AND COMPANY, Indianapolis, Indiana 46285 (US)
(72) Inventor: Morris, Tommy Clifford, Indianapolis, Indiana 46205 (US); Lange, Hans Joerg, 20144 Hamburg (DE)
(74) Representative: Hudson, Christopher Mark

(57) **Abstract**

A pharmaceutically elegant solid oral formulation comprising olanzapine as an active ingredient with one or more pharmaceutically acceptable excipients is provided, wherein the olanzapine is coated with cetyl alcohol, cetyl esters wax, carnauba wax, shellac, beeswax, magnesium stearate, hydroxypropyl methyl cellulose, hydroxyethyl cellulose, methylhydroxyethylcellulose, sodium carboxymethylcellulose, hydroxypropylcellulose, polyvinyl pyrrolidone, dimethylaminoethyl methacrylatemethylacrylate acid ester copolymer, ethylacrylate-methylmethacrylate copolymer, methylcellulose, and ethylcellulose, to prevent dissolvation of olanzapine.

## Description

This invention provides an improved pharmaceutically elegant formulation of 2-methyl-4-(4-methyl-1-piperazinyl)-10H-thieno[2,3-b] [1,5] benzodiazepine, hereinafter referred to as olanzapine, and processes for the preparation thereof.

Olanzapine has shown great promise in the treatment of psychotic patients and is currently being evaluated for that purpose. Certain tablet formulations of olanzapine are known, as described in U.S. Patent No. 5,229,382. However, improved oral formulations were desired in light of the moisture sensitive, metastable nature of olanzapine, the tendency of olanzapine to undesirably discolor in the known tablet formulation, that is the formulation disclosed in 5,229,382, and due to the surprisingly potent nature of olanzapine. A pharmaceutically elegant granule or microparticle formulation was especially desired. Such granule formulation was particularly challenging in light of the exacerbating effect of surface contact with ambient air and moist environments and the relatively large surface area inherent in a granule formulation.

The discoloration and mottled appearance does not produce an increase in the number of total related substances; however, the color change and appearance is not generally considered pharmaceutically desirable. The color change could be particularly undesirable for patients suffering from a psychotic conditions. Indeed, the patient most likely to receive olanzapine is a patient suffering from hallucinations, delusions, and loss of touch with reality. Thus, a formulation having consistent color and appearance is most desired.

Applicants have discovered that directly coating the olanzapine substance with one or more carefully selected polymers can provide drug material that is resistant to such discoloration when formulated as a granule. Additionally, the presently claimed invention is useful for tablet formulations wherein a tablet subcoating is undesirable.

The presently claimed invention provides a pharmaceutically elegant solid oral formulation comprising olanzapine as an active ingredient with one or more pharmaceutically acceptable excipients,
wherein the olanzapine is coated with a polymer selected from the group consisting of cetyl alcohol, cetyl esters wax, carnauba wax, shellac, beeswax, magnesium stearate, hydroxypropyl methyl cellulose, hydroxyethyl cellulose, methylhydroxyethylcellulose, sodium carboxymethylcellulose, hydroxypropylcellulose, polyvinyl pyrrolidone, dimethylaminoethyl methacrylatemethylacrylate acid ester copolymer, ethylacrylate-methylmethacrylate copolymer, methylcellulose, and ethylcellulose.

It is particularly preferred that the olanzapine coat does not contain polyethylene glycol.

It is especially desired that the solid oral formulation is a granule. It may be desired that the formulation is a microparticle.

Olanzapine, a potent compound showing promising activity for use in treating psychotic patients, tends to be metastable, undergo pharmaceutically undesired discoloration, and demands care to assure homogeniety of the finished solid formulation.

Applicants have discovered that olanzapine undergoes undesirable discoloration when contacted with certain excipients including powder blends. Further, the discoloration is exacerbated by ambient air conditions, at elevated temperatures, and by moist environments.

Although the discoloration phenomenon does not produce an increase in the number of total related substances, the browning and mottling appearance is not generally considered pharmaceutically acceptable for commercial purposes. Further, the discoloration is particularly disturbing when a tablet formulation is administered to a psychotic patient, which patient may be especially troubled by the changing appearance of their medication.

The discoloration phenomenon is particularly troublesome for a granule formulation. Such formulation inherently exposes more olanzapine to ambient or humid conditions by virtue of the increased outer surface area relative to a solid tablet formulation. The present invention provides the desired pharmaceutically elegant granule formulation.

Applicants have discovered that coating the olanzapine compound with a polymer selected from the group consisting of cetyl alcohol, cetyl esters wax, carnauba wax, shellac, beeswax, magnesium stearate, hydroxypropyl methyl cellulose, hydroxyethyl cellulose, methylhydroxyethylcellulose, sodium carboxymethylcellulose, hydroxypropylcellulose, polyvinyl pyrrolidone, dimethylaminoethyl methacrylatemethylacrylate acid ester copolymer, ethylacrylate-methylmethacrylate copolymer, methylcellulose, and ethylcellulose; as a coating or subcoating provides a uniform, physical stability and effectively prevents the undesired discoloration phenomenon in the formulation. The formulation is most preferredly in a tablet form; however, granule formulation and the like are desired as well.

Most preferred polymer coats are hydroxypropyl methyl cellulose, hydroxypropylcellulose, methylcellulose, and ethylcellulose. An especially preferred polymer coat is hydroxypropyl methylcellulose.

It is especially preferred that the formulation contain the most stable anhydrous form of olanzapine, referred to herein as Form II; (see EP 733,635) however, other forms of olanzapine are contemplated. Form II has a typical x-ray powder diffraction pattern as represented by the following interplanar spacings:

| **d** |
|---|
| 10.2689 |
| 8.577 |
| 7.4721 |
| 7.125 |
| 6.1459 |
| 6.071 |
| 5.4849 |
| 5.2181 |
| 5.1251 |
| 4.9874 |
| 4.7665 |
| 4.7158 |
| 4.4787 |
| 4.3307 |
| 4.2294 |
| 4.141 |
| 3.9873 |
| 3.7206 |
| 3.5645 |
| 3.5366 |
| 3.3828 |
| 3.2516 |
| 3.134 |
| 3.0848 |
| 3.0638 |
| 3.0111 |
| 2.8739 |
| 2.8102 |
| 2.7217 |
| 2.6432 |
| 2.6007 |

A typical example of an x-ray diffraction pattern for Form II is as follows wherein d represents the interplanar spacing and I/I₁ represents the typical relative intensities:

| **d** | **I/I**_{**1**} |
|---|---|
| 10.2689 | 100.00 |
| 8.577 | 7.96 |
| 7.4721 | 1.41 |
| 7.125 | 6.50 |
| 6.1459 | 3.12 |
| 6.071 | 5.12 |
| 5.4849 | 0.52 |
| 5.2181 | 6.86 |
| 5.1251 | 2.47 |
| 4.9874 | 7.41 |
| 4.7665 | 4.03 |
| 4.7158 | 6.80 |
| 4.4787 | 14.72 |
| 4.3307 | 1.48 |
| 4.2294 | 23.19 |
| 4.141 | 11.28 |
| 3.9873 | 9.01 |
| 3.7206 | 14.04 |
| 3.5645 | 2.27 |
| 3.5366 | 4.85 |
| 3.3828 | 3.47 |
| 3.2516 | 1.25 |
| 3.134 | 0.81 |
| 3.0848 | 0.45 |
| 3.0638 | 1.34 |
| 3.0111 | 3.51 |
| 2.8739 | 0.79 |
| 2.8102 | 1.47 |
| 2.7217 | 0.20 |
| 2.6432 | 1.26 |
| 2.6007 | 0.77 |

The x-ray diffraction patterns set out herein were obtained using a Siemens D5000 x-ray powder diffractometer having a copper Kₐ radiation source of wavelength, l =1·541Å.

The formulation of the invention preferredly contains substantially pure Form II as the active ingredient.

As used herein "substantially pure" refers to Form II associated with less than about 5% undesired polymorphic form of olanzapine (herein referred to as "Undesired Form"), preferably less than about 2% Undesired Form, and more preferably less than about 1% Undesired Form. Further, "substantially pure" Form II will contain less than about 0.5% related substances, wherein "related substances" refers to undesired chemical impurities or residual organic solvent. In particular, "substantially pure" Form II preferably contain less than about 0.05% ccntent of acetonitrile, more preferably, less than about 0.005% content of acetonitrile.

As used herein, the term "mammal" shall refer to the Mammalia class of higher vertebrates. The term "mammal" includes, but is not limited to, a human. The term "treating" as used herein includes prophylaxis of the named condition or amelioration or elimination of the condition once it has been established.

Form II is the most stable anhydrous form of olanzapine known and is therefore important for the commercial development of pharmaceutically elegant formulations. Olanzapine may form an undesired crystal form in the presence of certain solvents and excipients, therefore, in making the compositions of the invention it is most desired to prepare the formulation using a method which does not require dissolution of the olanzapine substance. The desired Form II can be converted to less desirable polymorphic forms by contact with methylene chloride, for example. Additionally, for example, polyethylene glycol contact with the olanzapine substance produces undesired discoloration, particularly under moist conditions.

### Stability Studies

Uncoated tablets stored at ambient conditions (approximately 23°C and 40% relative humidity) in amber, high density polyethylene bottles do not show signs of discoloration after 24 months; however, if the bottle is opened such that the tablets are exposed to open air ambient conditions then discoloration occurs within 5 days.

A new solid oral formulation is prepared using olanzapine that is coated with hydroxypropropyl methylcellulose. The new formulation is monitored for 90 days of open dish storage at 40°C, 60°C, 40°C/75 %RH, ambient temperature with 75% RH, and at ambient temperature with 85% RH. The hydroxypropyl methylcellulose coating which is free of polyethylene glycol is much preferred to ensure that discoloration does not occur. It provides an effective barrier between the olanzapine drug substance and excipients which might otherwise cause discoloration. For example, the hydroxypropylmethylcellulose coating provides sufficient barrier to prevent discoloration attributable to polyethylene glycol which is commonly found in certain pharmaceutical expients and coatings. Thus the hydroxypropyl methylcellulose coated olanzapine is a surprising and important component of pharmaceutically elegant solid oral formulations of olanzapine.

The coating of the olanzapine drug substance can be completed using techniques familiar to the artisan. For example:

### 1. SPRAY DRYING.

The mixture of olanzapine and polymer in a solvent is sprayed into a stream of hot air in a conventional manner. This causes the solvent to evaporate and the powder is collected in the spray drying vessel.

### 2. EXTERNAL LIQUID PHASE.

The mixture of olanzapine and polymer, which is in the form of a solution or suspension, is poured into a liquid external phase. The liquid external phase comprises a solvent which is immiscible or partially immiscible with the olanzapine/polymer mixture.

Following the addition of the olanzapine/polymer mixture to the external liquid phase, the two phase mixture obtained is emulsified, for example by rapid mixing. The emulsion formed may be either stable or unstable. The solvent may be removed by a number of ways familiar to the artisan. For example, but not limited to, passive removal (evaporation during mixing), heating, rotary film evaporator, vacuum with or without heating, microwave drying with or without vacuum, freeze drying, and the like.

The artisan can select a desired formulation for the coated olanzapine using techniques familiar to the skilled artisan. For example:

A diluent or bulking agent should be selected to provide an increase in tablet size. The artisan can utilize known methods to select a bulking agent which provides hardness, friability, and disintegration time that is satisfactory for pharmaceutical usage. The bulking agent should be selected to provide a tablet that has characterstics desired by the patient as well as comply with applicable regulatory guidelines.

One especially preferred diluent or bulking agent is lactose. Various forms of lactose are appropriate for such formulations including anhydrous, hydrous, and spray dried forms. The most desired form of lactose can be selected based on desired dissolution, content uniformity, hardness, friability, and disintegration time. The skilled artisan is aware of the regulatory requirements for hardness, friability, and disintegration time and can adjust the diluent or bulking agents using known techniques to achieve the desired physical characteristics.

The formulation should include a binder for use in the granulation step. The artisan can choose an appropriate binder based on the acceptable viscosity, and desired hydration. Hydroxypropyl cellulose is especially preferred for use as a binder in the granulation step. The hydroxypropyl cellulose may vary in particle size. Fine grade hydroxypropyl cellulose is especially preferred for most claimed formulations.

The desired formulation includes a disintegrant for use in the granulation as well as in the running powders to facilitate the disintegration process. There are a variety of grades available, and the grade may be selected based on the acceptable batch variability. A particularly prefered disintegrant is crospovidone. A fine grade of crospovidone provides particularly desirable consistency between batches.

The artisan may choose appropriate dry binders using known methods. Such binders should be selected to assure that satisfactory friability is attained. Most preferably, dry binder is microcrystalline cellulose; however, other appropriate dry binders may be selected. Such microcrystalline cellulose may be in a granular form.

The artisan can choose an appropriate lubricant to prevent sticking and picking of the tablets to the compression tooling. One preferred lubricant is magnesium stearate.

The artisan can readily choose other appropriate aqueous dispersion film coats (color mix) for application over the hydroxypropyl methylcellulose layer. Typically, the color mixture is a dry blend of ingredients which may be dispersed in water and used as an aqueous dispersion to film coat solid formulations. One example of a typical color mixture is comprised of hydroxypropyl methylcellulose, polyethylene glycol, polysorbate 80, and titianium dioxide.

A variety of edible inks known to the artisan are appropriate for imprinting the finished formulation. For example, one typical edible ink is comprised of shellac, ehtyl alcohol, isopropyl alcohol, n-butyl alcohol, propylene glycol, ammonium hydroxide, and FD&C Blue.

The solid formulation is most preferably prepared using hydroxypropyl methylcellulose coated olanzapine. The solid formulation may be polished using standard methods such as carnauba wax polishing, if desired.

Olanzapine is effective over a wide dosage range, the actual dose administered being dependent on the condition being treated. For example, in the treatment of adult humans, dosages of from about 0.25 to 50 mg, preferably from 1 to 30 mg, and most preferably 1 to 25 mg per day may be used. A once a day dosage is normally sufficient, although divided doses may be administered. For treatment of central nervous system disorders, a dose range of from 1 to 30 mg, preferably 1 to 25 mg per day is suitable. Radiolabelled Form II 2-methyl-4-(4-methyl-1-piperazinyl)-10H-thieno-[2,3-b] [1,5]benzodiazepine, can be detected in the saliva and thus the compound can potentially be monitored in patients to assess compliance.

A preferred formulation of the invention is a solid oral formulation comprising from about 1 to about 25 mg olanzapine as an active ingedient, wherein such solid oral formulation is prepared using olanzapine that is coated with hydroxypropyl methylcellulose. Especially preferred is an oral formulation comprising from 1 to 25 mg of anhydrous Form II olanzapine as an effective amount of the active ingredient, provided that such Form II is coated with hydroxypropyl methylcellulose.

Most preferably, the solid oral formulation is contained in packaging materials which protect the formulation from moisture and light. For example, suitable packaging materials include amber colored high density polyethylene bottles, amber colored glass bottles, and other containers made of a material which inhibits the passage of light. Most preferably, the packaging will include a desiccant pack. The container may be sealed with an aluminum foil blister to provide the desired protection and maintain product stability.

The materials for the present invention can be purchased or prepared by a variety of procedures well known to those of ordinary skill in the art. Olanzapine can be prepared as described by Chakrabarti in U.S. Patent No 5,229,382 ('382), herein incorporated by reference in its entirety. It is most desirable to prepare a rapidly dissolving formulation comprising substantially pure crystalline Form II. Such substantially pure crystalline Form II olanzapine may be prepared using the techniques described herein by the Preparation section herein *infra.*

Compound characterization methods include, for example, x-ray powder pattern analysis, thermogravimetric analysis (TGA), differential scanning calorimetery (DSC), titrametric analysis for water, and H¹-NMR analysis for solvent content.

The formulations are studied to assure that the Form II polymorph is substantially pure using ¹³C Cross polarization / magic angle spinning (CP/MAS) NMR. Spectra were obtained using a Varian Unity 400 MHz spectrometer operating at a carbon frequency of 100.577 MHz and equipped with a complete solids accessory and Varian 5 mm or 7 mm VT CP/MAS probes. Measurement conditions were optimized for Olanzapine Form II and were as follows: 90° proton r.f. pulse 4.5 ms, contact time 1.1 ms, pulse repetition time 5 s, MAS frequency 7.0 kHz, spectral width 50 kHz, and acquisition time 50 ms. Chemical shifts were referenced to the CH₃ of hexamethylbenzene (d = 17.3 ppm) by sample replacement. Therefore, the formulations of this invention provide substantially pure Form II planzapite polymorph in a pharmaceutically elegant formulation without producing undesired polymorphic transformation.

The following examples are provided for purposes of illustration and are not to be construed as limiting the scope of the claimed invention.

### Preparation 1

### Technical Grade olanzapine

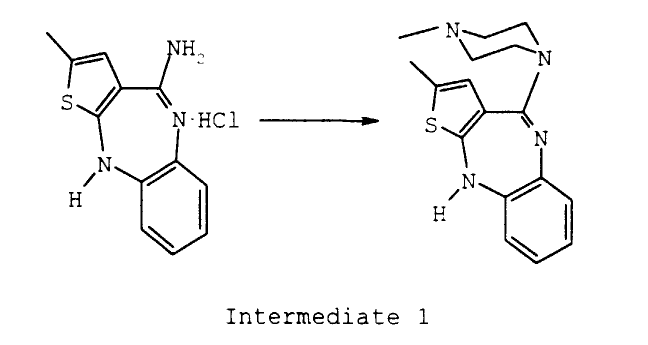

In a suitable three neck flask the following was added:

| | |
|---|---|
| Dimethylsulfoxide (analytical) | 6 volumes |
| Intermediate 1 | 75 g |
| N-Methylpiperazine (reagent) | 6 equivalents |

Intermediate 1 can be prepared using methods known to the skilled artisan. For example, the preparation of the Intermediate 1 is taught in the '382 patent.

A sub-surface nitrogen sparge line was added to remove the ammonia formed during the reaction. The reaction was heated to 120°C and maintained at that temperature throughout the duration of the reaction. The reactions were followed by HPLC until 5% of the intermediate 1 was left unreacted. After the reaction was complete, the mixture was allowed to cool slowly to 20°C (about 2 hours). The reaction mixture was then transferred to an appropriate three neck round bottom flask and water bath. To this solution with agitation was added 10 volumes reagent grade methanol and the reaction was stirred at 20°C for 30 minutes. Three volumes of water was added slowly over about 30 minutes. The reaction slurry was cooled to zero to 5°C and stirred for 30 minutes. The product was filtered and the wet cake was washed with chilled methanol. The wet cake was dried in vacuo at 45°C overnight. The product was identified as technical olanzapine.
Yield: 76.7%; Potency: 98.1%

### Preparation 2

### Form II

A 270 g sample of technical grade 2-methyl-4-(4-methyl-1-piperazinyl)-10H-thieno[2,3-b] [1,5]benzodiazepine was suspended in anhydrous ethyl acetate (2.7 L) . The mixture was heated to 76°C and maintained at 76°C for 30 minutes. The mixture was allowed to cool to 25°C. The resulting product was isolated using vacuum filtration. The product was identified as Form II using x-ray powder analysis.
Yield: 197 g.

The process described above for preparing Form II provides a pharmaceutically elegant product having potency ≥ 97%, total related substances < 0.5% and an isolated yield of > 73%.

### EXAMPLE 1

| Olanzapine 5.0 mg Tablets (Fluidized bed granulation process) | |
|---|---|
| **Names of Ingredients** | **Quantity (mg/tablet)** |
| **Active Ingredient** | |
| Olanzapine (coated) | 5.50 |
| **Other Ingredients** | |
| Lactose | 155.50 |
| Hydroxypropyl Cellulose | 8.00 |
| Crospovidone | 10.00 |
| Microcrystalline Cellulose | 20.00 |
| Magnesium Stearate | 1.00 |

The hydroxypropyl cellulose is dissolved in purified water to form a solution for granulation. The coated olanzapine is added to a fluidized bed granulator/dryer along with other excipients, the diluent (lactose) , an a portion of the disintegrant (crospovidone). This mixture is granulated in the fluidized bed granulator/dryer with the hydroxypropyl cellulose solution and subsequently dried. The dried material may be appropriately sized and then added to a blender.

The outside powders consisting of microcrystalline cellulose (granular), magnesium stearate , and the remainder of the crospovidone are added to the granulation. The mixture is blended and compressed with the appropriate tooling on tablet compression equipment.

### EXAMPLE 2

| Olanzapine 20.0 mg Granules (Fluidized bed granulation process) | |
|---|---|
| **Names of Ingredients** | **Quantity (mg/dose)** |
| **Active Ingredient** | |
| Olanzapine (coated) | 22.00 |

| **Other Ingredients** | |
|---|---|
| Mannitol | 928.00 |
| Hydroxypropyl methylcellulose | 50.00 |

The hydroxypropyl methylcellulose was dissolved in purified water to form a solution for granulation. The coated olanzapine is added to a fluidized bed granulator/dryer along with the diluent (mannitol). This mixture is granulated in the fluidized bed granulator/dryer with the hydroxypropyl cellulose solution and subsequently dried. The dried material may be appropriately sized and packaged as a granule formulation.

### EXAMPLE 3

| Olanzapine 20.0 mg Tablets (direct compression process) | |
|---|---|
| **Names of Ingredients** | **Quantity (mg/tablet)** |
| **Active Ingredient** | |
| Olanzapine (coated) | 22.00 |
| **Other Ingredients** | |
| Lactose | 232.12 |
| Hydroxypropyl Cellulose | 13.00 |
| Crospovidone | 16.25 |
| Microcrystalline Cellulose | 40.00 |
| Magnesium Stearate | 1.63 |

The coated olanzapine is added to a blender along with other excipients, the diluent (mannitol), disintegrant (crospovidone), binders (hydroxypropyl cellulose and microcrystalline cellulose), and lubricant (magnesium stearate). This mixture is blended and compressed with the appropriate tooling on tablet compression equipment.

## Claims

1. A solid oral formulation comprising olanzapine as an active ingredient with one or more pharmaceutically acceptable excipients,
wherein the olanzapine is coated with a polymer selected from the group consisting of cetyl alcohol, cetyl esters wax, carnauba wax, shellac, beeswax, magnesium stearate, hydroxypropyl methyl cellulose, hydroxyethyl cellulose, methylhydroxyethylcellulose, sodium carboxymethylcellulose, hydroxypropylcellulose, polyvinyl pyrrolidone, dimethylaminoethyl methacrylatemethylacrylate acid ester copolymer, ethylacrylate-methylmethacrylate copolymer, methylcellulose, and ethylcellulose.

2. A formulation as claimed by **Claim 1** wherein the polymer is selected from the group consisting of hydroxypropyl methyl cellulose, hydroxypropylcellulose, methylcellulose, and ethylcellulose.

3. A formulation as claimed by **Claim 2** wherein the polymer is hydroxypropylmethyl cellulose.

4. A formulation as claimed by **Claim 1** wherein the polymer is free of propylene glycol.

5. A formulation as claimed by **Claim 4** wherein formulation is a granule.

6. A formulation as claimed by **Claim 1** wherein the solid formulation is a tablet.

7. A formulation as claimed by **Claim 1** wherein the solid formulation is agrannule.

8. A formulation as claimed by **Claim 5** wherein each granule provides a dose of olanzapine selected from the group consisting of 1, 2.5, 5, 7.5, 10, 15, 20 and 25 mg.

9. A formulation as claimed by **Claim 6** wherein each tablet provides a dose of olanzapine selected from the group consisting of 1, 2.5, 5, 7.5, 10, 15, 20 and 25 mg.

10. A solid formulation as claimed by **Claim 1** for use in treating a condition selected from the group consisting of psychosis, schizophrenia, a schizophriform disorder, mild anxiety, a gastrointestinal disorder, and acute mania.

11. A formulation as claimed by **Claim 1** wherein olanzapine is substantially pure Form II polymorph having a typical x-ray powder diffraction pattern as represented by the following interplanar spacings:
| **d (A)** |
|---|
| 10.2689 |
| 8.577 |
| 7.4721 |
| 7.125 |
| 6.1459 |
| 6.071 |
| 5.4849 |
| 5.2181 |
| 5.1251 |
| 4.9874 |
| 4.7665 |
| 4.7158 |
| 4.4787 |
| 4.3307 |
| 4.2294 |
| 4.141 |
| 3.9873 |
| 3.7206 |
| 3.5645 |
| 3.5366 |
| 3.3828 |
| 3.2516 |
| 3.134 |
| 3.0848 |
| 3.0638 |
| 3.0111 |
| 2.8739 |
| 2.8102 |
| 2.7217 |
| 2.6432 |
| 2.6007 |
